# EUROPEAN PATENT SPECIFICATION

(11) **EP 2 694 155 B1**
(45) Date of publication and mention of the grant of the patent: **14.10.2015**
(21) Application number: 12748523.3
(22) Date of filing: 02.04.2012
(51) Int. Cl.: A61N 1/04

(54) **TEXTILE ELECTRODE DEVICE FOR ACQUISITION OF ELECTROPHYSIOLOGICAL SIGNALS FROM THE SKIN AND MANUFACTURING PROCESS THEREOF**
TEXTILELEKTRODENVORRICHTUNG ZUR ERFASSUNG ELEKTROPHYSIOLOGISCHER SIGNALE AUS DER HAUT UND HERSTELLUNGSVERFAHREN DAFÜR
DISPOSITIF À ÉLECTRODE TEXTILE POUR L'ACQUISITION DE SIGNAUX ÉLECTROPHYSIOLOGIQUES À PARTIR DE LA PEAU ET PROCÉDÉ DE FABRICATION ASSOCIÉ

(30) Priority: 01.04.2011 IT TO20110297
(43) Date of publication of application: 12.02.2014
(73) Proprietor: Politecnico Di Torino, 10129 Torino (IT)
(72) Inventor: GAZZONI, Marco, 10144 Torino (IT); MERLETTI, Roberto, 10124 Torino (IT)
(74) Representative: Nannucci, Lorenzo
(86) International application number: PCT/IB2012/001133
(87) International publication number: WO 2012/140522

(56) References cited:
- EP-A1- 1 457 552
- WO-A1-2009/043196
- DE-U1-202006 007 226
- FR-A1- 2 493 184
- US-A1- 2006 211 934
- US-A1- 2010 004 720
- US-B2- 7 426 415

## Description

### TECHNICAL FIELD

The present invention relates to a textile electrode device for the acquisition of electrophysiological signals from the skin and an associated manufacturing process.

### BACKGROUND ART

In various fields, in the medical field or in sports for example, there is a known need to detect and acquire bioelectric (or electrophysiological, or biopotential) signals on the surface of the skin of a subject to be monitored.

For example, these signals can include surface electromyogram (EMG) signals, representative of the electrical activity generated during muscular contraction, electrocardiogram (ECG) signals, representative of the electrical activity associated with myocardial contraction during the cardiac cycle, or electroencephalogram (EEG) signals, representative of the extracellular current flow generated by the aggregate of cortical neuron activities.

Although having the considerable advantage of being totally non-invasive, detection of bioelectric signals on the surface of the skin has a series of problems, mainly related to the small amplitude of the signals to be recorded (due to the filter effect of the tissues interposed between the source of the electric potentials and the skin surface) and the difficulty in achieving a stable contact between detection sensors and the skin (for example, due to the presence of hairs on the skin).

The detection techniques for surface bioelectric signals contemplate placing on the skin (for example, in the case of EMG signal detection, on the portion of skin over the muscle or muscles of interest) of detection devices comprising one or more electrodes of an electrically conductive material, for example silver or gold, and the use of conductive gel for improving electrode-skin contact. The electrodes, arranged in arrays or matrices for example, are fixed on the skin by an adhesive or biadhesive material. The detected electrical signals are then sent to appropriate conditioning electronics for subsequent amplification and filtering operations.

The ever-increasing demand for technologies to monitor chronic conditions (in hospital or at home), to offer rehabilitation support and to provide welfare technologies for the elderly and disabled has highlighted the limits of traditional systems, in particular with regard to the need for preparing the skin, skin irritation related to the use of gel and to the difficulty of using these systems for long-term monitoring.

Electrodes that do not require the use of gel at the electrode-skin interface enable the above-specified limits to be overcome. For example, among the solutions for simplifying and improving the detection of surface bioelectric signals, the use of textile electrode devices has been proposed, i.e. composed of electrically conductive filaments woven into a textile, using known sewing or embroidering techniques.

These electrode devices have the advantage of being easy to position on the surface of the skin when integrated in a garment. However, the electrical contact between these devices and the skin is often found to be critical, with high impedance values on the electrode-skin contact (and therefore with associated deterioration in the quality of the acquired signals) and instability of the contact.

US-A1-2006/211934 discloses a textile-based electrode, including a fabric portion having stretch-recovery non-conductive yarns and an electrically conductive region having stretch-recovery electrically conductive yarn filaments. When incorporated into a garment, the electrodes can be used to monitor biophysical characteristics, such as the garment wearer's heart rate.

### DISCLOSURE OF INVENTION

The object of the present invention is that of providing a textile electrode device and an associated manufacturing process that enable the known acquisition techniques of surface bioelectric signals to be perfected and the previously highlighted problems to be solved, in full or in part.

According to the present invention, a textile electrode device and a related manufacturing process are thus provided, as defined in the appended claims.

### BRIEF DESCRIPTION OF THE DRAWINGS

For a better understanding of the present invention, some preferred embodiments will now be described, purely by way of a non-limitative example and with reference to the attached drawings, where:
- Figure 1 shows, in plan view, a textile electrode device according to one embodiment of the present invention;
- Figure 2 shows a section of the device in Figure 1, taken along the section line II-II;
- Figure 3 shows an enlarged portion of the section in Figure 2;
- Figure 4 shows, in plan view, a textile electrode device according to a further embodiment of the present invention;
- Figures 5 and 6 show axonometric views of the device in Figure 4 in operating conditions; and
- Figure 7 schematically shows an apparatus for manufacturing a textile electrode device according to a further aspect of the present invention.

### BEST MODE FOR CARRYING OUT THE INVENTION

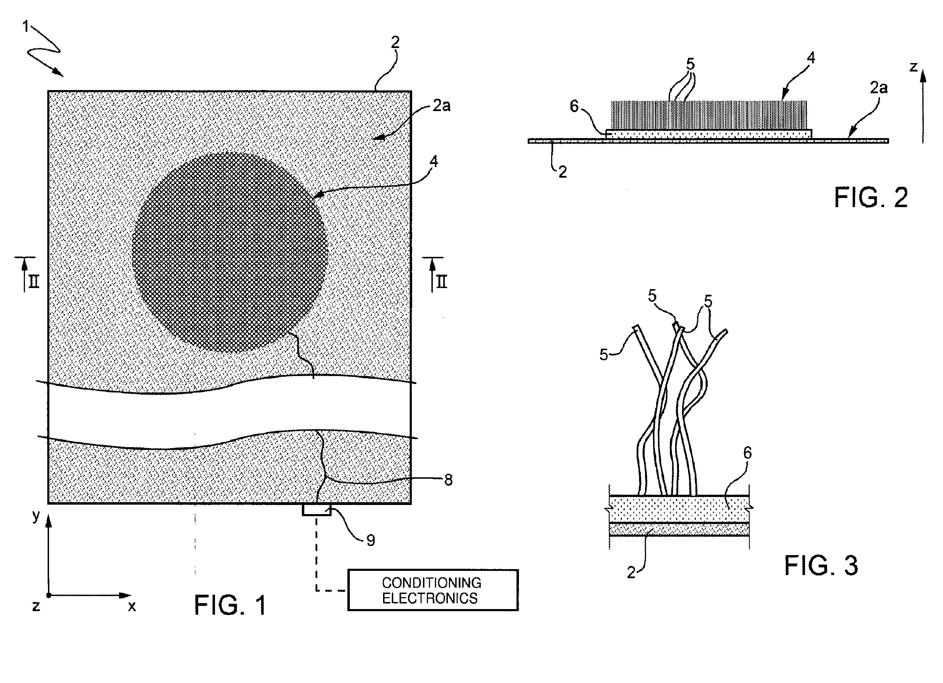

As shown in Figures 1-3, a textile electrode device 1, according to one embodiment of the present invention, comprises a substrate 2, for example a preferably elastic textile substrate (such as cotton or Lycra for example), and at least one detection electrode 4, arranged on the substrate 2, for detection of electrophysiological signals (in particular for detecting biopotentials).

According to one particular aspect of the present invention, the detection electrode 4 is made using the flocking technique; this technique enables, in a per-se known manner, the deposition of a plurality of small fibres (so-called flock) on a substrate, these being arranged in a direction generally orthogonal to the substrate.

The detection electrode 4, as shown in Figures 2 and 3 in particular, therefore comprises a plurality of textile fibres 5, of which a certain number are conductive, such as polyamide or acrylic fibres coated in metal (silver, gold or otherwise); the fibres 5 are arranged in a direction substantially orthogonal to the substrate 2 (i.e. along a z-axis orthogonal to an x-y plane defining an upper surface 2a of the substrate 2).

Thanks to the flocking process, the fibres 5 are arranged in close contact one with the other and at least partially interwoven with each other (for example, with a "comb pattern" configuration), so as to make mutual electrical contact; in particular, this mutual electrical contact between the various fibres 5 guarantees substantially uniform electrical conductivity across the entire surface of the detection electrode 4.

The fibres 5 have, for example, a length of 0.5-2 mm (along the above-mentioned z-axis) and a linear density of 1-20 dtex.

An adhesive layer 6, of conductive or non-conductive material, formed for example by a wash-resistant glue (elastic or rigid after drying), is interposed between the fibres 5 and the substrate 2 and, in particular, enables the fastening and anchoring of the fibres 5 to the substrate 2; in detail, one end of the fibres 5, close to the substrate 2, is buried and incorporated into the adhesive layer 6.

The textile electrode device 1 also comprises a conducting wire 8, formed for example by bare metal (copper or steel), natural or artificial textile yarn (nylon or cotton) coated with a conductive material (copper or silver), or conductive polymers, mechanically and electrically connected to the detection electrode 4.

The conducting wire 8 is preferably interwoven in the substrate 2, for example by sewing, embroidering or weaving techniques in general, so as to maintain the elasticity of the substrate 2 if it is elastic (for example, by using an elastic stitch if the conducting wire is sewn, or an elastic conducting wire, obtained for example with a metal wire spun on an elastic textile yarn, if inserted in the weft and warp).

The conducting wire 8 extends to an electrical connector 9, designed to interface with the conditioning electronics 10 (schematically shown in Figure 1), to enable the processing of the surface bioelectric signals detected by the textile electrode device 1 (when opportunely arranged on a portion of the skin, with the detection electrode 4 placed in contact with the skin).

For example, the conducting wire 8 may have a core of conducting material sheathed with an insulating material. In this case, the insulating material is conveniently removed at one or more anchorage points of an end of the conducting wire 8 at the detection electrode 4; this end is connected to the detection electrode 4, for example by means of a conductive glue or sewing.

For example, the electrical connector 9 may be arranged externally to the substrate 2, flush with, and at an outer edge of, the same substrate 2, so as to be free to interface with the conditioning electronics 9 (in a per-se known manner).

The Applicant has verified that, after positioning the textile electrode device 1 on the skin of a subject, it is possible to acquire electric biopotentials on the skin with excellent electrode-skin contact assured by the special arrangement of the fibres 5 (thanks to the properties of the flocking process, especially the one using the electrostatic technique). Given the large number of fibres 5 mutually in contact with each other, of which the detection electrode 4 is formed, and given their arrangement substantially orthogonal to the skin (in a comb pattern for example), which enables any hairs to be accommodated in the space between the fibres 5, the possible presence of hairs on the skin does not cause problems.

Document FR 2 493 184 A1 describes an electrode intended for use in electrostatic applications in various application domains, such as for example air or water filtration and purification, and generally having the purpose of enabling the discharge of electrostatic charges. The electrode is formed by a plurality of fibres, which permit mechanical particle filtering and creating a constant electrostatic field and uniform ionization even over wide surfaces, avoiding the so-called "point effect" associated with traditional electrodes, where the charges are concentrated on the perimeter.

Among the various fields of application, this document cites the so-called "bioélectronique" (bioelectronics); however, it is known that this term refers to natural medicine systems, aimed at the discharge and elimination (so-called "earthing") of accumulated electrostatic charges, which clearly have no connection with the acquisition of electrophysiological signals on the skin of a subject.

In particular, the above-mentioned document FR 2 493 184 A1 does not provide any teaching regarding the possible application of the electrode on the skin of a subject, and still less on the use of the electrode itself as a detection instrument for electrophysiological signals and for their subsequent processing by electronic signal-processing devices.

In general, the Applicant has verified for the first time that the use of a detection electrode made with the flocking technique within the field of detection of electrophysiological signals from the skin of a subject, enables a plurality of specific technical advantages to be obtained, which are not achievable with, nor immediately foreseeable when starting from traditional solutions (for example, by means of Ag or AgCl electrodes, or traditional textile electrodes); these advantages including:
- increase in the contact surface between electrode and skin, with the consequent lowering of the electrode-skin impedance and contact noise;
- optimal adaptation to the irregular surface of the skin;
- overcoming the obstacle represented by the presence of hairs on the skin; and
- improved comfort for the subject, thanks to the "velvety" effect offered by the flocked material.

According to a further aspect of the present invention, a first plurality of fibres 5 of the detection electrode 4 is composed of a conductive textile, while a second plurality of fibres 5 of the same detection electrode 4 is composed of a so-called "super-absorbent" material; the various fibres 5 are mutually arranged in the detection electrode 4 in a manner substantially similar to what previously discussed, with the conductive fibres interposed and mixed with fibres of super-absorbent material.

In the known manner, super-absorbent materials, such as super-absorbent polymers for example, are materials of high hygroscopic capacity, capable of absorbing amounts of liquids hundreds of times greater than their weight.

Advantageously, the introduction of fibres of super-absorbent material permits keeping the contact with the skin damp and maintaining optimal electrode-skin contact over time, avoiding, for example, evaporation of the water used to reduce electrode-skin impedance.

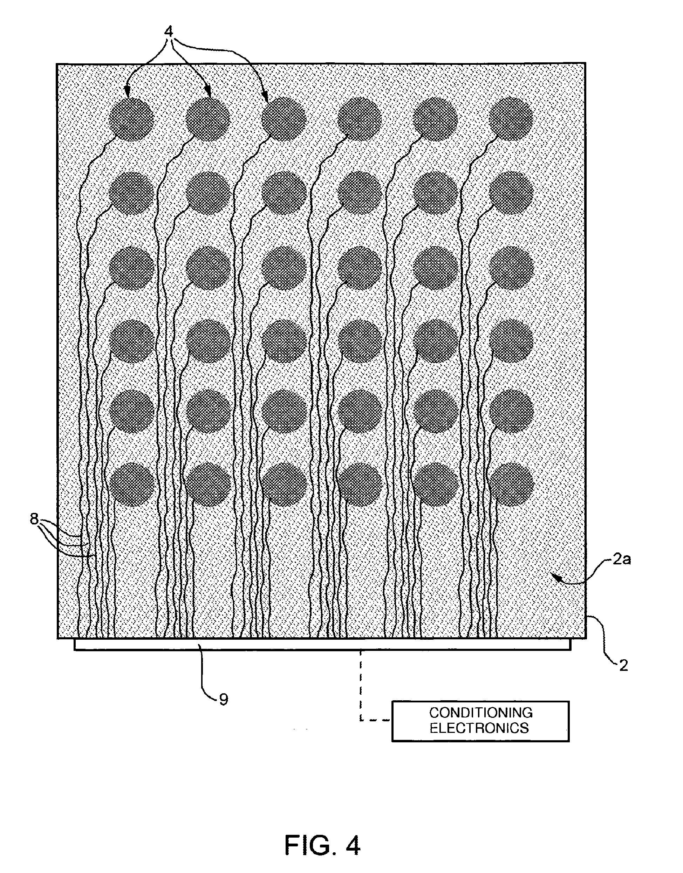

Figure 4 shows a further embodiment of the textile electrode device, again indicated by reference numeral 1 and comprising, in this case, a plurality of detection electrodes 4, made with the flocking technique as previously described and arranged on the substrate 2 according to a regular square matrix layout (with six rows and columns in the example shown).

A regular and distributed arrangement of the detection electrodes 4 enables detection of the spatial distribution of electric potential on the skin and is, for example, advantageous for realizing acquisition systems for high-density surface EMG (HD-EMG) signals.

In this case, the detection electrodes 4 (having a circular shape with a diameter of 1 cm in the example shown) are arranged at regular distances on the substrate 2 (for example, with an inter-electrode distance of 2 cm) and a respective conducting wire 8 is associated with each one of them for carrying the detected bioelectric signals. The various conducting wires 8, which again are advantageously interwoven in the substrate 2, pass through the substrate 2 to reach a single electrical connector, again indicated by reference numeral 9, which houses their ends for electrical connection with the conditioning electronics 9.

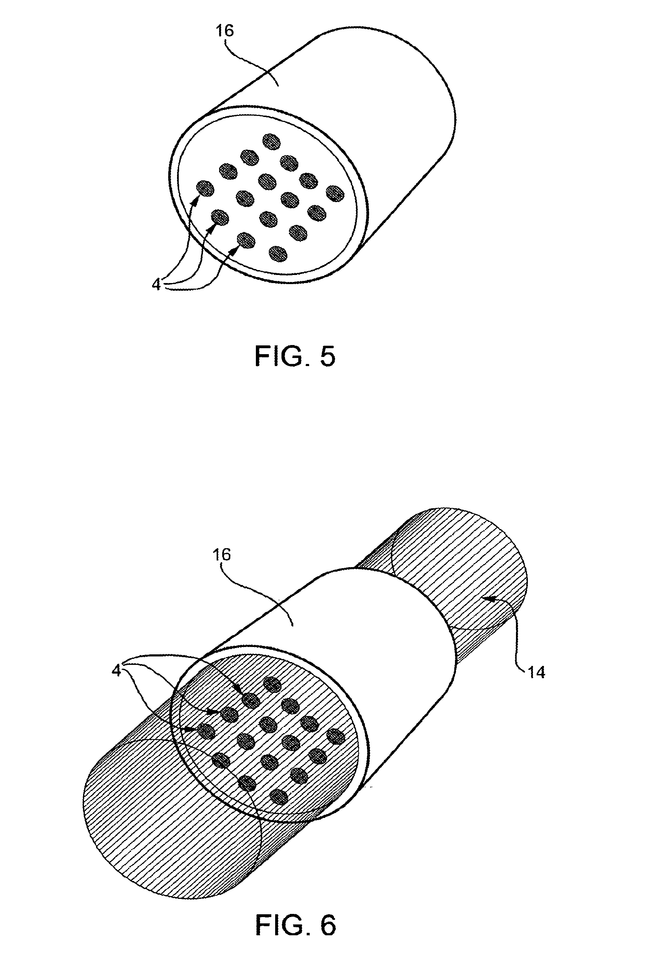

Figures 5 and 6 show, by way of non-limitative example, an exemplary usage of the textile electrode device 1, in this case applied to a limb 14 for detecting surface EMG signals.

In this case, the substrate 2 is part of (or carried by) an elastic sheath 16, having a hollow cylindrical shape; the detection electrodes 4 are arranged on an inner lateral surface of the aforementioned elastic sheath 16. In use, the elastic sheath 16 is placed around the limb 14, over a muscle to be examined, so that the detection electrodes 4 are arranged in contact with the skin of the limb 14 (note that the substrate 2 is arranged parallel to and around the skin surface). Advantageously, the elastic sheath 16 is placed on the limb 14 in a firm and stable manner, so as to enable detection of the bioelectric signals in a continuative manner over time, with uniform electrical performance.

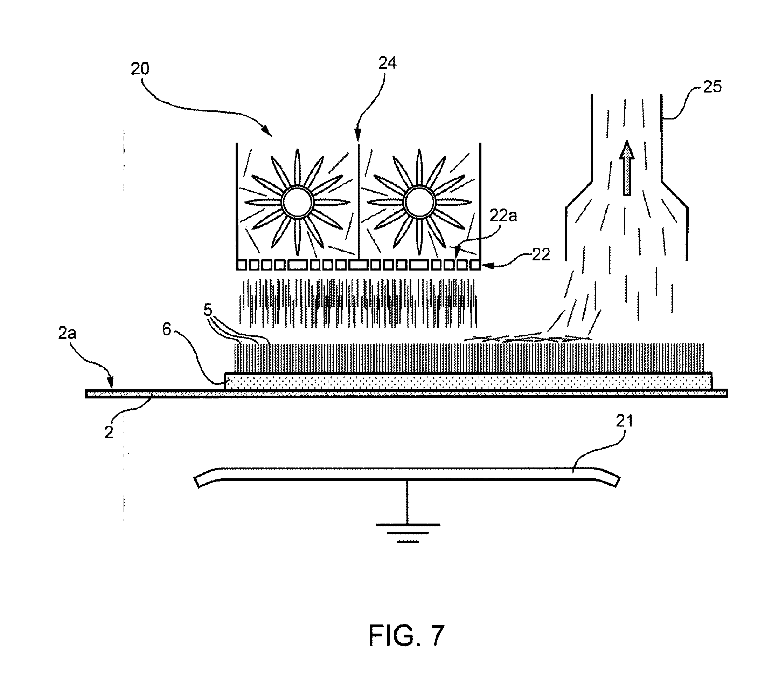

A flocking apparatus, indicated by reference numeral 20, for making detection electrodes 4 on the substrate 2, in particular by means of the electrostatic flocking technique, will now be described, with particular reference to Figure 7.

In a manner in itself known (this technique is currently used in the textile field for making surfaces with a surface aspect similar to suede, cloth or velvet for clothing or furnishing fabrics), electrostatic flocking uses an electrical charge sufficient to orientate the fibres 5 and induce alignment perpendicular to the substrate 2.

In particular, the adhesive layer 6 must first be placed on the substrate 2, the former being opportunely shaped and defined, in particular according to the arrangement that is required to be assumed by the detection electrodes 4 on the substrate 2. Advantageously, the adhesive layer 6 is applied on the substrate 2 by means of a frame (using a silk-screen technique), opportunely shaped so as to allow laying only on the areas that will be subsequently occupied by the detection electrodes 4.

It should be noted that the selective deposition of the adhesive layer 6 only on the detection areas enables achieving considerable precision and reproducibility in the realization of the detection electrodes 4 (as described in the following), guaranteeing improved characteristics with respect to textile electrodes obtained with traditional weaving techniques (for example, embroidery or sewing).

The substrate 2, on which an opportunely shaped adhesive layer 6 is already present, is then subjected to a high-intensity electrostatic field inside the flocking apparatus 20. In particular, this field is generated between two electrodes: an earth electrode 21 and an electrode grid 22, set at a high voltage (the presence of apertures 22a may be noted between the electrodes of the electrode grid 22).

A plurality of fibres 5 (the conductive flock and possibly the super-absorbent flock) is released in the same area inside the flocking apparatus 20, for example, by allowing the fibres 5 to fall towards the substrate 2 under the effect of gravity from a hopper, or other container 24, placed above the electrode grid 22 (the fibres 5 thus reaching the substrate 2 through the apertures 22a in the electrode grid 22).

The electrostatic field charges the various fibres 5, which thus become aligned with the direction of the electrostatic field lines (substantially in a direction orthogonal to the substrate 2) and are attracted towards the earth electrode 21; in this way, the fibres 5 come into contact with the adhesive layer 6, remaining incorporated therein and adhering to the substrate 2 in a stable manner. In particular, the electrostatic application ensures that the fibres 5 become arranged substantially orthogonal to the upper surface 2a of the substrate 2, in a dense manner and in mutual contact at the detection areas.

The flocking apparatus 20 may also comprise a suction duct 25, for conveniently removing the excess fibres (flock).

From examination of the characteristics of the device according to the present invention, the advantages that it provides are evident.

In particular, the textile electrode device 1 enables the electrical potential on the skin to be detected without requiring the use of conductive gel, or the interposition of other conductive materials, thanks to the excellent electrode-skin contact assured by the particular arrangement of the fibres 5 (thanks to the properties of the flocking process); the possible presence of hairs on the skin does not cause problems in this case. The presence of super-absorbent fibres, inserted between the conductive fibres further improves the quality of the contact, especially for prolonged acquisitions.

Therefore, it is also possible to record bioelectric signals for long periods, without problems of skin irritation and with substantially constant electrical performance over time.

The detection electrodes 4 are simple and inexpensive to manufacture, even on an industrial scale, while it is also easy to define their number and arrangement on the substrate 2. In particular, through a single flocking process, it is possible to define a desired number of detection electrodes 4 on a same substrate 2 (this device therefore being well suited to the high-definition spatial monitoring of bioelectric signals).

Finally, it is understood that changes and modifications can be made to what described and illustrated herein without departing from the scope of the present invention, as defined in the appended claims.

In particular, it is evident that the size and shape of each detection electrode 4 (as well as the number of detection electrodes 4) may be freely chosen, for example, according to the characteristics of the bioelectric signal to be acquired or the portion of skin on which the detection electrode 4 is to be arranged (for example, circular, square, rectangular, or other geometrical shapes may be equally provided for the detection electrodes 4).

The actual materials constituting the textile electrode device 1 may be different with respect to what previously illustrated.

The substrate 2 could advantageously be part of, or carried by, sensor-fitted garments that can be worn by the subject for whom the measurements are to be taken (in a manner similar to what shown for the sheath 16 in Figure 5), so that the detection electrodes 4 are arranged in contact with the skin, close to the desired detection zone.

## Claims

1. A textile electrode device (1), comprising: a substrate (2), designed to be positioned on the skin of a subject; at least a first detection electrode (4) arranged on the substrate (2) and configured to detect electrophysiological signals from the skin of the subject; and at least a first conducting element (8), carried by the substrate (2) and electrically connected to the first detection electrode (4), **characterized in that** the first detection electrode (4) is obtained by means of the flocking technique and comprises a first plurality of textile fibres (5), of an electrically conductive material, arranged in a direction substantially orthogonal to an upper surface (2a) of the substrate (2) and reciprocally in contact with each other.

2. The device according to claim 1, wherein the first detection electrode (4) is obtained by means of the electrostatic flocking technique.

3. The device according to any of the preceding claims, wherein said first detection electrode (4) further comprises a second plurality of textile fibres (5), of a super-absorbent material, interposed between the textile fibres of conductive material of the first plurality.

4. The device according to any of the preceding claims, further comprising an adhesive layer (6) arranged between the substrate (2) and the first detection electrode (4), to which the textile fibres (5) are anchored.

5. The device according to any of the preceding claims, wherein the textile fibres (5) have a length in the range between 0.5 mm and 2 mm, and a linear density in the range between 1 dtex and 20 dtex.

6. The device according to any of the preceding claims, wherein said first detection electrode (4) is obtained by means of the flocking technique, and also comprising further detection electrodes (4), these also obtained by the flocking technique and arranged over the substrate (2), and further conducting elements (8), each one electrically connected to a respective detection electrode (4).

7. The device according to any of the preceding claims, wherein the first conducting element (8) is woven through the substrate (2), and has a first end in electrical and mechanical contact with the first detection electrode (4) and a second end in contact with an electrical connector element (9), designed for connection to a conditioning electronics (10) for the detected electrophysiological signals.

8. The device according to any of the preceding claims, wherein the substrate (2) comprises an elastic fabric.

9. The device according to claim 8, wherein the substrate (2) is part of, or is carried by, a wearable element that can be worn by the subject, so that the first detection electrode (4) is directly in contact with the skin.

10. A sensor-fitted wearable element (16), comprising a textile electrode device (1) according to any of the preceding claims, the sensor-fitted wearable element (16) being designed to be worn by a subject for detection of electrophysiological signals from the skin.

11. A process for manufacturing a textile electrode device (1), comprising the steps of:
- providing a substrate (2), designed to be positioned on the skin of a subject;
- forming at least a first detection electrode (4) on the substrate (2), configured to detect electrophysiological signals from the skin of the subject; and
- forming at least a first conducting element (8), carried by the substrate (2) and electrically connected to the first detection electrode (4),
**characterized in that** the step of forming at least a first detection electrode comprises coupling to the substrate (5) a first plurality of textile fibres (2), of an electrically conductive material, arranged in a direction substantially orthogonal to an upper surface (2a) of the substrate (2) and reciprocally in contact with each other; wherein the step of forming at least a first detection electrode comprises:
- depositing an adhesive layer (6) on the substrate (2), shaped so as to define at least one detection area;
- directing a plurality of textile fibres (5) towards the substrate (2), by means of the flocking technique, so that they adhere to the adhesive layer (6) at the detection area.

12. The process according to claim 11, wherein the plurality of textile fibres (5) comprises fibres of an electrically conductive material, and wherein the step of directing towards the substrate comprises generating an electrostatic field on the substrate (2) so as to orient the textile fibres (5) towards the substrate (2), in a direction substantially orthogonal to an upper surface (2a) of the substrate (2).

13. The process according to claim 12, wherein the plurality of textile fibres (5) also comprises fibres of a super-absorbent material, interposed between the fibres of conductive material.

14. The process according to any of claims 11-13, wherein the step of forming at least a first detection electrode (4) also comprises the step of forming further detection electrodes (4) arranged over the substrate (2), through the steps of:
- shaping the adhesive layer (6) over the substrate (2) so as to define further detection areas, one for each further detection electrode (4); and
- directing the plurality of textile fibres (5) towards the substrate (2), so that they adhere to the adhesive layer (6) at the detection areas, thereby forming the further detection electrodes (4).

## Patentansprüche

1. Eine Textilelektrodenvorrichtung (1) aufweisend: Ein Substrat (2), das zur Auflage auf die Haut einer Person bestimmt ist; wenigstens eine erste Detektionselektrode (4), die auf dem Substrat angeordnet ist und ausgebildet ist, um elektrophysiologische Signale von der Haut der Person zu detektieren; und wenigstens ein erstes leitendes Element (8), das von dem Substrat (2) getragen wird und elektrisch mit der ersten Detektionselektrode (4) verbunden ist, **dadurch gekennzeichnet, dass** die erste Detektionselektrode (4) mittels der Beflockungstechnik hergestellt ist und eine erste Vielzahl von textilen Fasern (5) aus einem elektrisch leitfähigen Material aufweist, die in einer Richtung im Wesentlichen orthogonal zu einer oberen Oberfläche (2a) des Substrats (2) angeordnet sind und wechselseitig miteinander in Kontakt sind.

2. Die Vorrichtung nach Anspruch 1, wobei die erste Detektionselektrode (4) mittels der elektrostatischen Beflockungstechnik hergestellt ist.

3. Die Vorrichtung nach einem der vorhergehenden Ansprüche, wobei die erste Detektionselektrode (4) ferner eine zweite Vielzahl von textilen Fasern (5) aus einem superabsorbierenden Material aufweist, die zwischen den textilen Fasern des leitenden Materials der ersten Vielzahl eingefügt sind.

4. Die Vorrichtung nach einem der vorhergehenden Ansprüche, ferner aufweisend eine adhäsive Schicht (6), die zwischen dem Substrat (2) und der ersten Detektionselektrode (4), mit der die textilen Fasern (5) verankert sind, angeordnet ist.

5. Die Vorrichtung nach einem der vorhergehenden Ansprüche, wobei die textilen Fasern (5) eine Länge haben, die in einem Bereich von 0,5 mm und 2 mm liegt, und eine lineare Dichte haben, die zwischen 1 dtex und 20 dtex liegt.

6. Die Vorrichtung nach einem der vorhergehenden Ansprüche, wobei die erste Detektionselektrode (4) mittels der Beflockungstechnik hergestellt ist, und auch weitere Detektionselektroden (4) aufweist, die auch mittels der Beflockungstechnik hergestellt und über das Substrat (2) angeordnet sind, und weitere leitende Elemente (8), die jeweils mit einer entsprechenden Detektionselektrode (4) elektrisch verbunden sind.

7. Die Vorrichtung nach einem der vorhergehenden Ansprüche, wobei das erste leitende Element (8) mit dem Substrat (2) verwoben ist und ein erstes Ende in elektrischem und mechanischem Kontakt mit der ersten Detektionselektrode (4) und ein zweites Ende in Kontakt mit einem elektrischen Leitungselement (9) hat, das zur Verbindung mit einer Aufbereitungselektronik (10) für die elektrophysiologischen Signale ausgebildet ist.

8. Die Vorrichtung nach einem der vorhergehenden Ansprüche, wobei das Substrat (2) ein elastisches Gewebe aufweist.

9. Die Vorrichtung nach Anspruch 8, wobei das Substrat (2) Teil von oder getragen von einem tragbaren Element ist, das von der Person getragen werden kann, so dass die erste Detektionselektrode (4) direkt mit der Haut in Kontakt ist.

10. Ein sensor-eingebautes tragbares Element (16) aufweisend eine Textilelektrodenvorrichtung (1) nach einem der vorhergehenden Ansprüche, wobei das sensor-eingebaute tragbare Element (16) ausgebildet ist, um von einer Person zur Detektion von elektrophysiologischen Signalen von der Haut getragen zu werden.

11. Ein Verfahren zur Herstellung einer Textilelektrodenvorrichtung (1), aufweisend die Schritte:
- Vorsehen eines Substrats (2), das geeignet ist, auf die Haut einer Person aufgelegt zu werden;
- Formen wenigstens einer ersten Detektionselektrode (4) auf dem Substrat (2), die zur Detektion von elektrophysiologischen Signalen von der Haut der Person ausgebildet ist; und
- Formen wenigstens eines ersten leitenden Elements (8), das von dem Substrat (2) getragen wird und elektrisch mit der ersten Detektionselektrode (4) verbunden ist, **dadurch gekennzeichnet, dass** der Schritt des Formens wenigstens einer ersten Detektionselektrode das Verbinden einer ersten Vielzahl von textilen Fasern (2) aus einem elektrisch leitenden Material mit dem Substrat (4) aufweist, die in einer Richtung im Wesentlichen orthogonal zu einer oberen Oberfläche (2a) des Substrats (2) angeordnet sind und wechselweise miteinander in Kontakt sind; wobei der Schritt des Formens wenigstens einer ersten Detektionselektrode aufweist:
- Auflegen einer adhäsiven Schicht (6) auf das Substrat (2) in einer derartigen Form, dass wenigstens eine Detektionsfläche definiert wird;
- Richten einer Vielzahl von textilen Fasern (5) gegen das Substrat (2) mittels der Beflockungstechnik, so dass diese an der adhäsiven Schicht (6) in der Detektionsfläche haften.

12. Das Verfahren nach Anspruch 11, wobei die Vielzahl von textilen Fasern (5) Fasern aus einem elektrisch leitenden Material aufweisen, und wobei der Schritt des Richtens gegen das Substrat das Erzeugen eines elektrostatischen Feldes auf dem Substrat (2) aufweist, so dass sich die textilen Fasern (5) gegenüber dem Substrat (2) in einer Richtung ausrichten, die im Wesentlichen orthogonal zu einer oberen Oberfläche (2a) des Substrats (2) ist.

13. Das Verfahren nach Anspruch 12, wobei die Vielzahl der textilen Fasern (5) auch Fasern aus einem superabsorbierenden Material aufweist, die zwischen den Fasern aus leitendem Material eingefügt sind.

14. Das Verfahren nach einem der Ansprüche 11 bis 13, wobei der Schritt des Formens wenigstens einer ersten Detektionselektrode (4) auch den Schritt des Formens weiterer über das Substrat (2) angeordneter Detektionselektroden (4) mit folgenden Schritten aufweist:
- Formen der adhäsiven Schicht (6) über das Substrat (2) derart, dass diese weitere Detektionsflächen definiert, eine für jede weitere Detektionselektrode (4); und
- Richten der Vielzahl von textilen Fasern (5) gegen das Substrat (2), so dass diese an der adhäsiven Schicht (6) in den Detektionsflächen haften, wodurch die weiteren Detektionselektroden (4) geformt werden

## Revendications

1. Dispositif d'électrode textile (1), comprenant : un substrat (2), conçu pour être positionné sur la peau d'un sujet ; au moins une première électrode de détection (4) agencée sur le substrat (2) et configurée pour détecter des signaux électrophysiologiques à partir de la peau du sujet ; et au moins un premier élément conducteur (8), supporté par le substrat (2) et connecté électriquement à la première électrode de détection (4),
**caractérisé en ce que** la première électrode de détection (4) est obtenue au moyen de la technique de flocage et comprend une première pluralité de fibres textile (5), en un matériau électriquement conducteur, agencées dans une direction sensiblement orthogonale à une surface supérieure (2a) du substrat (2) et réciproquement en contact les unes avec les autres.

2. Dispositif selon la revendication 1, dans lequel la première électrode de détection (4) est obtenue au moyen de la technique de flocage électrostatique.

3. Dispositif selon l'une quelconque des revendications précédentes, dans lequel ladite première électrode de détection (4) comprend en outre une deuxième pluralité de fibres textile (5), en un matériau super absorbant, interposées entre les fibres textile en un matériau conducteur de la première pluralité.

4. Dispositif selon l'une quelconque des revendications précédentes, comprenant en outre une couche adhésive (6) agencée entre le substrat (2) et la première électrode de détection (4), dans laquelle les fibres textile (5) sont ancrées.

5. Dispositif selon l'une quelconque des revendications précédentes, dans lequel les fibres textile (5) ont une longueur dans la plage entre 0,5 mm et 2 mm, et une densité linéaire dans la plage entre 1 dtex et 20 dtex.

6. Dispositif selon l'une quelconque des revendications précédentes, dans lequel ladite première électrode de détection (4) est obtenue au moyen de la technique de flocage, et comprenant également des électrodes de détection (4) supplémentaires, celles-ci étant également obtenues par la technique de flocage et agencées sur le substrat (2), et des éléments conducteurs (8) supplémentaires, chacun étant connecté électriquement à une électrode de détection (4) respective.

7. Dispositif selon l'une quelconque des revendications précédentes, dans lequel le premier élément conducteur (8) est tissé à travers le substrat (2), et a une première extrémité en contact électrique et mécanique avec la première électrode de détection (4) et une deuxième extrémité en contact avec un élément de connexion électrique (9), conçu pour une connexion à l'électronique de conditionnement (10) pour les signaux électrophysiologiques détectés.

8. Dispositif selon l'une quelconque des revendications précédentes, dans lequel le substrat (2) comprend un tissu élastique.

9. Dispositif selon la revendication 8, dans lequel le substrat (2) fait partie de, ou est supporté par, un élément portable qui peut être porté par le sujet, de sorte que la première électrode de détection (4) soit directement en contact avec la peau.

10. Elément portable équipé d'un capteur (16), comprenant un dispositif d'électrode textile (1) selon l'une quelconque des revendications précédentes, l'élément portable équipé d'un capteur (16) étant conçu pour être porté par un sujet pour la détection de signaux électrophysiologiques à partir de la peau.

11. Processus pour la fabrication d'un dispositif d'électrode textile (1), comprenant les étapes :
- de fourniture d'un substrat (2), conçu pour être positionné sur la peau d'un sujet ;
- de formation d'au moins une première électrode de détection (4) sur le substrat (2), configurée pour détecter des signaux électrophysiologiques à partir de la peau du sujet ; et
- de formation d'au moins un premier élément conducteur (8), supporté par le substrat (2) et connecté électriquement à la première électrode de détection (4),
**caractérisé en ce que** l'étape de formation d'au moins une première électrode de détection comprend le couplage au substrat (5) d'une première pluralité de fibres textile (2), en un matériau électriquement conducteur, agencées dans une direction sensiblement orthogonale à une surface supérieure (2a) du substrat (2) et réciproquement en contact les unes avec les autres ; dans lequel l'étape de formation d'au moins une première électrode de détection consiste à :
- déposer une couche adhésive (6) sur le substrat (2), formée de manière à définir au moins une zone de détection ;
- diriger une pluralité de fibres textile (5) vers le substrat (2), au moyen de la technique de flocage, de manière à ce qu'elles adhèrent à la couche adhésive (6) au niveau de la zone de détection.

12. Processus selon la revendication 11, dans lequel la pluralité de fibres textile (5) comprennent des fibres en un matériau électriquement conducteur, et dans lequel l'étape consistant à les diriger vers le substrat comprend la génération d'un champ électrostatique sur le substrat (2) de manière à orienter les fibres textile (5) vers le substrat (2), dans une direction sensiblement orthogonale à une surface supérieure (2a) du substrat (2).

13. Processus selon la revendication 12, dans lequel la pluralité de fibres textile (5) comprend également des fibres en un matériau super absorbant, interposées entre les fibres en un matériau conducteur.

14. Processus selon l'une quelconque des revendications 11 à 13, dans lequel l'étape de formation d'au moins une première électrode de détection (4) comprend également l'étape de formation d'électrodes de détection (4) supplémentaires agencées sur le substrat (2), par les étapes consistant à :
- mettre en forme la couche adhésive (6) sur le substrat (2) de manière à définir des zones de détection supplémentaire, une pour chaque électrode de détection (4) supplémentaire ; et
- diriger la pluralité de fibres textile (5) vers le substrat (2), de sorte qu'elles adhèrent à la couche adhésive (6) au niveau des zones de détection, formant de ce fait les électrodes de détection (4) supplémentaires.
